# EUROPÄISCHE PATENTSCHRIFT

(11) **EP 0 657 414 B1**
(45) Veröffentlichungstag und Bekanntmachung des Hinweises auf die Patenterteilung: **22.10.1997**
(21) Anmeldenummer: 94118584.5
(22) Anmeldetag: 25.11.1994
(51) Int. Cl.: C07C 68/02

(54) **Verfahren zur Herstellung von Dialkyldicarbonaten**
Process for preparing dialkyl dicarbonates
Procédé de préparation de dicarbonates de dialcoyle

(30) Priorität: 08.12.1993 DE 4341747
(43) Veröffentlichungstag der Anmeldung: 14.06.1995
(73) Patentinhaber: BAYER AG, 51368 Leverkusen (DE)
(72) Erfinder: Pies, Michael, Dr., D-47249 Duisburg (DE); Fiege, Helmut, Dr., D-51373 Leverkusen (DE); Käsbauer, Josef, Dr., D-42929 Wermelskirchen (DE); Merz, Gebhard, Dr., D-47798 Krefeld (DE)

(56) Entgegenhaltungen:
- EP-A- 0 468 404
- DE-A- 1 418 849
- TETRATHEDRON, Bd.44, Nr.9, 1988 D. PLUSQUELLEC ET AL. 'A new Synthesis of Carboxylic and Carbonic Acid anhydrides using Phase Transfer Reactions'

## Beschreibung

Die vorliegende Erfindung betrifft ein Verfahren zur Herstellung von Dialkyldicarbonaten durch Umsetzung von entsprechenden Chlorameisensäureestern mit Alkalien in Gegenwart spezieller Katalysatoren. Dialkyldicarbonate werden z.B. als Kaltsterilisationsmittel zum Entkeimen von Fruchtsaftgetränken und alkoholfreiem Wein eingesetzt. Dialkyldicarbonate werden auch als Dialkylpyrocarbonate bezeichnet.

Es ist schon ein Verfahren zur Herstellung von u.a. Dialkyldicarbonaten bekannt geworden, bei dem man Chlorameisensäureester mit wäßriger Natronlauge in Gegenwart eines organischen Lösungsmittels und in Gegenwart von Tetra-n-butylammoniumchlorid oder Tri-n-caprylmethylammoniumchlorid als Phasentransferkatalysator umsetzt (siehe Tetrahedron 44 (9), 2471-2476 (1988)). Nachteilig bei diesem Verfahren ist, daß es hohe Mengen an Katalysatoren benötigt (0,1 Äquivalent = 10 Mol-%) und, daß der Katalysator nur sehr schwierig wiedergewinnbar und recyclisierbar ist, weil er sich entweder in der wäßrigen oder in der organischen Phase ansammelt und daraus nur mit großem Aufwand abgetrennt werden kann. Eine Wiedergewinnung und Recyclisierung des Katalysators ist zum einen aus Kostengründen sehr erwünscht, zum anderen auch deshalb, weil sonst Probleme mit der Entsorgung katalysatorhaltiger Produktionstückstände entstehen.

Es wurde nun ein Verfahren zur Herstellung von Dialkyldicarbonaten aus entsprechenden Halogenameisensäureestern durch Umsetzung mit Alkalien in Gegenwart von mit Wasser nicht mischbaren organischen Lösungsmitteln und in Gegenwart von Katalysatoren gefunden, das dadurch gekennzeichnet ist, daß man als Katalysatoren Benzylalkyldimethylammoniumhalogenide einsetzt.

Mit dem erfindungsgemäßen Verfahren kann man beispielsweise aus Halogenameisensäureestern der Formel (I) 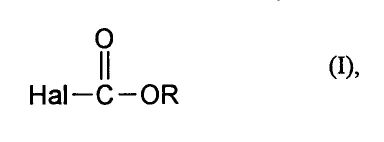 in der
- Hal: für Halogen, insbesondere Chlor, und
- R: für einen geradkettigen oder verzweigten C₁-C₂₀-Alkylrest stehen,
Dialkyldicarbonate der Formel (II) herstellen 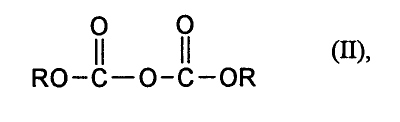 in der
- R: die bei Formel (I) angegebene Bedeutung hat.

In den Formeln (I) und (II) steht R vorzugsweise für einen C₁-C₈-Alkylrest, und das an den Sauerstoff gebundene C-Atom der Alkylgruppe weist vorzugsweise mindestens noch ein Wasserstoffatom auf. Insbesondere steht R für Methyl, Ethyl, n-Propyl, i-Propyl, n-Butyl oder i-Butyl.

Als Alkalien kommen insbesondere Alkalihydroxide in Frage, wie Natrium-und Kaliumhydroxid, die vorzugsweise in Form von wäßrigen Lösungen zum Einsatz gelangen. Beispielsweise kann man 5 bis 30 gew.-%ige wäßrige Alkalihydroxidlösungen verwenden. Bevorzugt sind 10 bis 20 gew.-%ige Lösungen.

Die Alkalien kann man beispielsweise in Mengen von 80 bis 120 Mol.-%, bezogen auf eingesetzten Halogenameisensäureestern einsetzen. Vorzugsweise liegt diese Menge im Bereich von 95 bis 105 Mol-%.

Als mit Wasser nicht mischbare organische Lösungsmittel kommen beispielsweise aromatische Kohlenwasserstoffe, chlorierte Kohlenwasserstoffe und mit Wasser nicht mischbare Ether in Frage. Bevorzugt sind Toluol, Xylol, Methylenchlorid und Diethylether, insbesondere Toluol und Methylenchlorid.

Das mit Wasser nicht mischbare organische Lösungsmittel kann man beispielsweise in Mengen von 30 bis 80 Gew.-%, bezogen auf den Halogenameisensäureester der Formel (I) einsetzen.

Es ist ein wesentliches Merkmal des erfindungsgemäßen Verfahrens, daß man als Katalysatoren Benzylalkyldimethylammoniumhalogenide einsetzt. Diese können beispielsweise der Formel (III) entsprechen 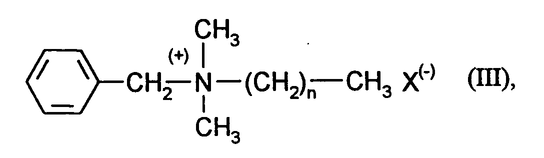 in der
- n: für eine ganze Zahl von 1 bis 20, vorzugsweise 8 bis 18, und
- X: für ein Halogen, vorzugsweise für Chlor
stehen. Man kann als Katalysator auch ein Gemisch verschiedener Individuen der Formel (III) einsetzen.

Den Katalysator kann man, bezogen auf Halogenameisensäureester, beispielsweise in einer Menge von 0,001 bis 0,1, vorzugsweise 0,005 bis 0,05, Mol-% einsetzen.

Das erfindungsgemäße Verfahren kann man bei Normaldruck, erhöhtem Druck oder niedrigem Druck durchführen.Vorzugsweise arbeitet man bei Normaldruck.

Die Reaktionstemperatur kann beispielsweise zwischen -10°C und der Siedetemperatur (bei Normaldruck) des eingesetzten Halogenameisensäureesters liegen. Vorzugsweise liegt sie im Bereich 0 bis 50°C.

Es ist vorteilhaft, während der Durchführung des erfindungsgemäßen Verfahrens kräftig zu rühren.

Das erfindungsgemäße Verfahren kann sowohl diskontinuierlich als auch kontinuierlich durchgeführt werden. Bei kontinuierlicher Arbeitsweise kann man beispielsweise mit Verweilzeiten zwischen 5 und 60 Minuten arbeiten. Bei diskontinuierlicher Arbeitsweise ist die Reaktion je nach der Größe des Ansatzes und der vorhandenen Kühlleistung im allgemeinen nach 10 Minuten bis 3 Stunden beendet.

Nach Durchführung des erfindungsgemäßen Verfahrens, gegebenenfalls nach Abkühlung und/oder der Zugabe von wenig Wasser, liegt ein 3-Phasensystem vor, nämlich eine organische Phase, die neben dem organischen Lösungsmittel im wesentlichen das hergestellte Dialkyldicarbonat und gegebenenfalls geringe Mengen von unumgesetztem Halogenameisensäureester enthält, eine wäßrige Phase, die neben Wasser die gebildeten anorganischen Salze enthält und eine Zwischenphase, in der sich der eingesetzte Katalysator und geringe Mengen des aus dem eingesetzten Halogenameisensäureessters durch Esterverseifung als Nebenprodukt gebildeten Alkohols befinden.

Aus der organischen Phase kann man das Reaktionsprodukt z.B. durch Entfernung des organischen Lösungsmittels durch Destillation gewinnen. Das organische Lösungsmittel kann gegebenenfalls recyclisiert werden. Die wäßrige Phase kann man, gegebenenfalls nach einer Vorreinigung, beispielsweise einer biologischen Klärstufe zur Entsorgung zuführen.

Es ist ein besonderer und überraschender Vorteil des erfindungsgemäßen Verfahrens, daß sich beim Ende der Reaktion praktisch der gesamte eingesetzte Katalysator in der Zwischenphase befindet. Die Zwischenphase macht volumenmäßig nur einen Bruchteil der wäßrigen und organischen Phase aus, weshalb sie den eingesetzten Katalysator schon in stark angereicherter Form enthält. Gegebenenfalls nach Zusatz von Wasser können durch Andestillieren der abgetrennten Zwischenphase bis zum Übergang von reinem Wasser die in der Zwischenphase enthaltenen Ausgangs- und flüchtigen Nebenprodukte abgetrennt werden. Die dann verbleibende wäßrige Lösung des Katalysators kann problemlos recyclisiert und beliebig häufig wiederverwendet werden. Die im gemäß dem Stand der Technik mit den dort verwendeten Katalysatoren auftretenden Abtrennungs-, Recyclisierungs- und/oder Entsorgungsprobleme treten beim erfindungsgemäßen Verfahren also nicht auf. Außerdem ist mit den erfindungsgemäß einzusetzenden Katalysatoren die Umsetzung in relativ kurzer Zeit durchzuführen.

### Beispiele

### Beispiel 1

Zu 139,4 g Chlorameisensäureisobutylester und 139,4 g Toluol wurde innerhalb von 35 Minuten eine Mischung aus 290 g 13,8 gew.-%iger wäßriger Natronlauge und 3,3 g Benzyldodecyldimethylammoniumchlorid getropft. Durch Kühlung wurde die Reaktionstemperatur zwischen 30 und 40°C gehalten. Nach beendeter Zugabe und Abschalten des Rührers trennte sich der Ansatz in drei Phasen. Die obere organische Phase enthielt 105,2 g Diisobutyldicarbonat (= 96,5 % der Theorie) und 0,32 g Chlorameisensäureisobutylester. In der Zwischenphase befanden sich der eingesetzte Katalysator und geringe Mengen von Isobutanol.

### Beispiel 2

Zu 100 g Chlorameisensäuremethylester und 100 g Toluol wurde innerhalb von 30 Minuten 291,5 g 13,8 gew.-%ige wäßrige Natronlauge und 3,3 g Benzyldodecyldimethylammoniumchlorid zudosiert. Die Reaktionstemperatur wurde durch Kühlung auf 10 bis 15°C gehalten. Nach beendeter Zugabe und Abstellen des Rührers wurden 100 ml Wasser zugegeben. Der Ansatz trennte sich in drei Phasen. In der obersten organischen Phase befanden sich 54,3 g Dimethyldicarbonat (= 85 % der Theorie) und 4,6 g Chlorameisensäuremethylester. In der Zwischenphase befand sich der eingesetzte Katalysator und geringe Mengen von Methanol.

### Beispiel 3

In einem doppelwandigen, gekühlten 60 ml-Konti-Rührreaktor wurden pro Stunde 240 ml 13,5 gew.-%ige wäßrige Natronlauge, die 1,7 Gew.-% Benzyl(C₈-C₁₈)-alkyl-dimethylammoniumchlorid enthielt und 278,4 ml einer 50 gew.-%igen Chlorameisensäureisobutylester/Toluol-Lösung eindosiert. Es ergab sich somit eine Verweilzeit von etwas mehr als 7 Minuten. Das ablaufende Reaktionsgemisch wurde in einen Abscheider geleitet, in dem es sich in drei Phasen trennte. Pro Stunde wurden in der oberen organischen Phase 98,1 g Diisobutyldicarbonat (98 % der Theorie) und 7,14 g unumgesetzten Chlorameisensäureisobutylester erhalten. Die Zwischenphase wurde auf 80 % ihres Volumens eingedämpft und der verbleibende Rückstand bei einer Wiederholung des Beispiels eingesetzt, bei der praktisch die gleichen Resultate erhalten wurden.

## Patentansprüche

1. Verfahren zur Herstellung von Dialkyldicarbonaten aus entsprechenden Halogenameisensäureestern durch Umsetzung mit Alkalien in Gegenwart von mit Wasser nicht mischbaren organischen Lösungsmitteln und in Gegenwart von Katalysatoren, dadurch gekennzeichnet, daß man als Katalysatoren Benzylalkyldimethylammoniumhalogenide einsetzt.

2. Verfahren nach Anspruch 1, dadurch gekennzeichnet, daß man aus Halogenameisensäureestern der Formel (I) 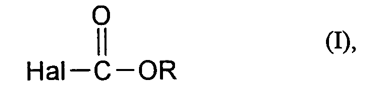 in der
Hal für Halogen und
R für einen geradkettigen oder verzweigten C₁-C₂₀-Alkylrest stehen,
Dialkyldicarbonate der Formel (II) herstellt 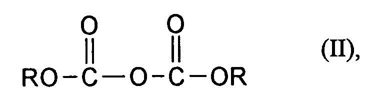 in der
R die bei Formel (I) angegebene Bedeutung hat.

3. Verfahren nach Ansprüchen 1 bis 2, dadurch gekennzeichnet, daß man als Alkalien Alkalihydroxide in Form wäßriger Lösungen einsetzt.

4. Verfahren nach Ansprüchen 1 bis 3, dadurch gekennzeichnet, daß man als mit Wasser nicht mischbare organische Lösungsmittel aromatische Kohlenwasserstoffe, chlorierte Kohlenwasserstoffe oder mit Wasser nicht mischbare Ether einsetzt.

5. Verfahren nach Ansprüchen 1 bis 4, dadurch gekennzeichnet, daß man als Benzylalkyldimethylammoniumhalogenide solche der Formel (III) einsetzt 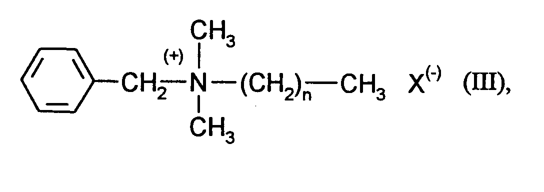 in der
n für eine ganze Zahl von 1 bis 20 und
X für ein Halogen stehen.

6. Verfahren nach Ansprüchen 1 bis 5, dadurch gekennzeichnet, daß man Benzylalkyldimethylammoniumhalogenide in einer Menge von 0,001 bis 0,1 Mol-%, bezogen auf Halogenameisensäureester, einsetzt.

7. Verfahren nach Ansprüchen 1 bis 6, dadurch gekennzeichnet, daß man es bei Reaktionstemperaturen zwischen -10°C und der Siedetemperatur (bei Normaldruck) des eingesetzten Halogenameisensäureesters durchführt.

8. Verfahren nach Ansprüchen 1 bis 7, dadurch gekennzeichnet, daß man nach der Beendigung der Reaktion die Zwischenphase abtrennt und nach der Abtrennung von Ausgangs- und flüchtigen Nebenprodukten aus der Zwischenphase den darin enthaltenen Katalysator zusammen mit Wasser abtrennt und recyclisiert.

## Claims

1. Process for the preparation of dialkyl dicarbonates from the corresponding halogenoformic esters by reaction with alkalis in the presence of water-immiscible organic solvents and in the presence of catalysts, characterized in that the catalysts used are benzylalkyldimethylammonium halides.

2. Process according to Claim 1, characterized in that from halogenoformic esters of the formula (I) 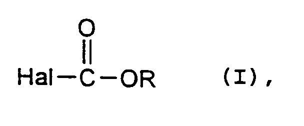 in which
Hal represents halogen, and
R represents a straight-chain or branched C₁-C₂₀-alkyl radical,
dialkyl dicarbonates of the formula (II) are prepared 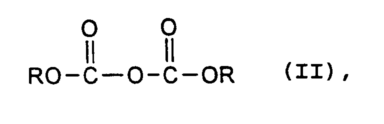 in which
R has the meaning given in formula (I).

3. Process according to Claims 1 to 2, characterized in that the alkalis used are alkali metal hydroxides in the form of aqueous solutions.

4. Process according to Claims 1 to 3, characterized in that the water-immiscible organic solvents used are aromatic hydrocarbons, chlorinated hydrocarbons or water-immiscible ethers.

5. Process according to Claims 1 to 4, characterized in that the benzylalkyldimethylammonium halides used are those of the formula (III) 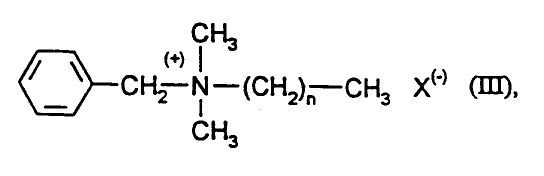 in which
n represents an integer from 1 to 20 and
X represents a halogen.

6. Process according to Claims 1 to 5, characterized in that benzylalkyldimethylammonium halides are used in an amount of 0.001 to 0.1 mol%, based on halogenoformic ester.

7. Process according to Claims 1 to 6, characterized in that the process is carried out at reaction temperatures between -10°C and the boiling temperature (at atmospheric pressure) of the halogenoformic ester used.

8. Process according to Claims 1 to 7, characterized in that after the reaction is completed, the intermediate phase is separated off and, after separating off starting products and volatile products from the intermediate phase, the catalyst contained therein is separated off together with water and recycled.

## Revendications

1. Procédé de préparation de dicarbonates de dialkyle à partir d'esters d'acide halogénoformique correspondants par réaction avec des alcalis en présence de solvants organiques non miscibles à l'eau et en présence de catalyseurs, caractérisé en ce que l'on utilise comme catalyseurs des halogénures de benzylalkyldiméthylammonium.

2. Procédé selon la revendication 1, caractérisé en ce que l'on prépare à partir d'esters d'acide halogénoformique de formule (I) : 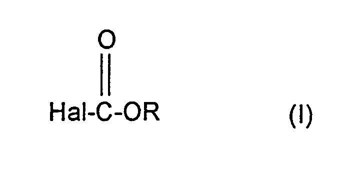 dans laquelle
Hal représente un halogène et
R représente un reste alkyle en C₁-C₂₀ linéaire ou ramifié,
des dicarbonates de dialkyle de formule (II): 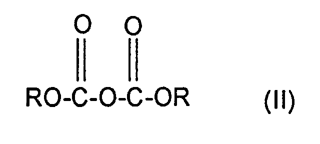 dans laquelle
R a la signification indiquée au sujet de la formule (I).

3. Procédé selon les revendications 1 et 2, caractérisé en ce que l'on utilise comme alcalis des hydroxydes alcalins sous forme de solutions aqueuses.

4. Procédé selon les revendications 1 à 3, caractérisé en ce que l'on utilise comme solvants organiques non miscibles à l'eau des hydrocarbures aromatiques, des hydrocarbures chlorés ou des éthers non miscibles à l'eau.

5. Procédé selon les revendications 1 à 4, caractérisé en ce que l'on utilise comme halogénures de benzylalkyldiméthylammonium des halogénures de formule (III): 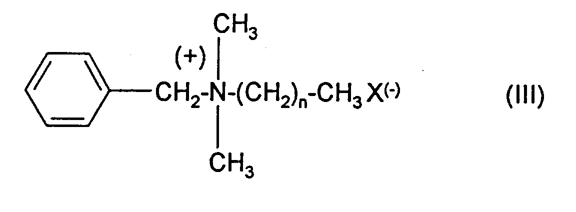 dans laquelle
n représente un nombre entier de 1 à 20 et
X représente un halogène.

6. Procédé selon les revendications 1 à 5, caractérisé en ce que l'on utilise des halogénures de benzylalkyldiméthylammonium en une quantité de 0,001 à 0,1 mol % par rapport aux esters d'acide halogénoformique.

7. Procédé selon les revendications 1 à 6, caractérisé en ce qu'on le met en oeuvre à des températures de réaction comprises entre -10°C et la température d'ébullition (à la pression normale) de l'ester d'acide halogénoformique utilisé.

8. Procédé selon les revendications 1 à 7, caractérisé en ce que, après la fin de la réaction, on sépare la phase intermédiaire et, après la séparation des produits initiaux et des sous-produits volatils d'avec la phase intermédiaire, on sépare et recycle le catalyseur qui y est contenu en même temps que l'eau.
